## Europäisches Patentamt

## European Patent Office

## Office européen des brevets

(19)

(11) **EP 0 766 959 B1**

(12) **FASCICULE DE BREVET EUROPEEN**

(45) Date de publication et mention
de la délivrance du brevet:
**25.11.1998 Bulletin 1998/48**

(51) Int. Cl.$^6$: **A61K 7/42**, A61K 7/48

(21) Numéro de dépôt: **96401933.5**

(22) Date de dépôt: **10.09.1996**

(54) **Compositions cosmétique photoprotectrices contenant des nanopigments de Tio2 et un acylaminoacide**

**Kosmetische Sonnenschutzmittel enthaltend TiO2 Nanopigmenten und eine Acylaminosäure**

**Cosmetic sunscreening compositions containing TiO2 nanopigments and an acrylaminoacid**

(84) Etats contractants désignés:
**AT BE CH DE DK ES FR GB GR IE IT LI LU MC NL PT SE**

(30) Priorité: **04.10.1995 FR 9511662**

(43) Date de publication de la demande:
**09.04.1997 Bulletin 1997/15**

(73) Titulaire: **L'OREAL**
**75008 Paris (FR)**

(72) Inventeurs:
• **Hansenne, Isabelle**
**75017 Paris (FR)**
• **Lety, Alain**
**77400 Lagny/s/Seine (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 679 382**    **WO-A-95/12381**

• **STN, Serveur de Bases de Données, XP002005227**
• **PATENT ABSTRACTS OF JAPAN vol. 13, no. 553 (C-663) & JP-A-01 228537 (AJINOMOTO CO.)**

**Description**

La présente invention concerne une nouvelle composition à usage topique plus particulièrement destinée à la photoprotection de la peau et/ou des cheveux contre le rayonnement ultraviolet ainsi que son utilisation dans le domaine cosmétique. Plus particulièrement, elle concerne une composition de couleur stable qui contient, dans un support cosmétiquement acceptable, au moins un nanopigment d'oxyde de titane et un acylaminoacide.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Pour filtrer les rayonnements UV-A et UV-B, il existe sur le marché différents types de filtres solaires : les pigments minéraux et les filtres organiques. Ces filtres doivent pouvoir absorber ou bloquer les rayons nocifs du soleil tout en restant inoffensifs pour l'utilisateur.

A ce titre, les pigments d'oxydes métalliques sont de plus en plus utilisés dans les produits solaires et les produits de jour, notamment de maquillage, compte tenu de leurs propriétés de diffusion et de réflexion des rayonnements UV qui leur confèrent un grand intérêt en terme de photoprotection : utilisés seuls, ils permettent d'obtenir une bonne protection contre les rayons UV ; associés à des filtres organiques, ils permettent de réaliser des produits hautement photoprotecteurs.

Ainsi, le pigment minéral le plus courant utilisé à ce jour est l'oxyde de titane, de préférence sous forme nanopigmentaire, dont les propriétés filtrantes sont bien connues.

Cependant, on observe une instabilité à la lumière des compositions contenant des pigments d'oxyde de titane dans un milieu sans oxygène, instabilité qui se manifeste par l'apparition d'une coloration bleue. Cette photocoloration, connue sous le nom de photobleuissement, n'est évidemment pas souhaitable d'un point de vue esthétique.

En vue de limiter ce phénomène de photoréactivité, on a proposé des pigments de $TiO_2$ traités en surface : ainsi, le brevet EP-B-0 461 130 décrit des nanoparticules de $TiO_2$ traitées par des anions phosphate De même, il est connu en cosmétique d'utiliser des pigments de $TiO_2$ traités en surface par de la silice ou de l'alumine. Cependant, ces traitements sont coûteux et difficiles à mettre en oeuvre.

Enfin, s'il est vrai que ces traitements de surface permettent de réduire la photoinstabilité des pigments d'oxydes métalliques, et en particulier le photobleuissement des formules contenant des pigments de $TiO_2$, cette diminution reste encore insuffisante.

Dans la demande de brevet français FR 95-07001 non encore publiée, la Demanderesse a proposé de résoudre le problème évoqué ci-dessus en encapsulant les oxydes métalliques à l'intérieur de vésicules lipidiques.

Une composition cosmétique comprenant une dispersion de microsphères et au moins un oxyde métallique est décrite dans la demande WO 95/12381. Un exemple de cette demande propose une composition pouvant comprendre, entre autres, de 1 à 10% de dioxyde de titane et de 0 à 10% de lauroyl lysine. Toutefois, cet exemple ne précise ni la taille de l'oxyde de titane employé ni l'effet éventuel de réduction du photobleuissement de cet oxyde de titane apporté par la lauroyl lysine.

Le document EP-A-0 679 382 décrit une poudre filtrant les rayonnements UV, cette poudre étant constituée de sphères de silice comprenant un matériau réflecteur des UV et traitées par de la N-Lauroyl-L-Lysine. Ce matériau réflecteur peut être un oxyde de titane. Un exemple de cette demande décrit une lotion comprenant, entre autres, un acylglutamate et une telle poudre filtrante constituée de sphères de silice préalablement recouvertes d'oxyde de titane sous forme de sol puis traitées par de la N-Lauroyl-L-Lysine. La taille du pigment d'oxyde de titane n'est pas précisée.

Le besoin subsiste donc quant à pouvoir disposer de compositions photostables contenant des pigments d'oxyde de titane tout en étant exemptes de vésicules protectrices. Au sens de la présente invention, on entend par photostable le fait qu'une composition donnée ne soit pas ou peu sujette au phénomène de photocoloration décrit ci-avant.

Ainsi, à la suite d'importantes recherches menées dans le domaine de la photoprotection évoqué ci-dessus, la Demanderesse a maintenant découvert que l'introduction, dans une composition contenant des pigments de $TiO_2$, d'au moins un lipide amphiphile ionique choisi parmi les acylaminoacides, et en particulier parmi les acylglutamates, réduisait significativement le phénomène de photobleuissement intrinsèquement attaché à cette composition.

Cette découverte est à la base de l'invention.

La présente invention a donc pour objet une composition cosmétique à usage topique, en particulier pour la photoprotection de la peau et/ou des cheveux, exempte de vésicules, et comprenant dans un support cosmétiquement acceptable, au moins un nanopigment d'oxyde de titane et au moins un lipide amphiphile ionique choisi parmi les acylaminoacides.

De façon avantageuse, le lipide amphiphile ionique est choisi parmi les acylglutamates.

La composition conforme à l'invention présente l'avantage d'être très peu sujette au photobleuissement.

Les compositions selon l'invention présentent en outre l'avantage d'être peu sujettes au blanchiment lorsqu'on les applique sur la peau. Enfin, ces compositions possèdent une grande douceur et un excellent facteur de protection solaire.

Un autre objet de l'invention est l'utilisation d'une composition telle que définie ci-dessus comme, ou pour la fabrication de, compositions cosmétiques destinées à la protection de la peau et/ou des cheveux contre le rayonnement ultraviolet, en particulier le rayonnement solaire.

Un autre objet de l'invention est un procédé de traitement cosmétique pour la protection de la peau et/ou des cheveux contre le rayonnement ultraviolet, en particulier le rayonnement solaire, consistant à appliquer sur ces derniers une quantité efficace d'une composition telle que définie ci-dessus.

L'invention a également pour objet l'utilisation d'un acylaminoacide, de préférence choisi parmi les acylglutamates, dans une composition contenant des pigments d'oxyde de titane, pour diminuer le photobleuissement dû à la présence desdits pigments.

L'invention a encore pour objet un procédé pour réduire le photobleuissement d'une composition cosmétique et/ou dermatologique contenant des pigments d'oxyde de titane, consistant à introduire dans ladite composition au moins un acylaminoacide, de préférence choisi parmi les acylglutamates.

D'autres caractéristiques, aspects et avantages de la présente invention apparaîtront à la lecture de la description détaillée qui va suivre.

Les lipides amphiphiles utilisés dans le cadre de la présente invention sont des lipides amphiphiles ioniques choisis parmi les acylaminoacides.

Selon un mode préféré de réalisation de l'invention, le lipide amphiphile ionique est choisi parmi les acylglutamates, et en particulier parmi les acylglutamates dont la chaîne acyle comprend de 10 à 30 atomes de carbone. De préférence, cette chaîne comprend de 10 à 20 atomes de carbone. Selon un mode préféré de réalisation de l'invention, les acylglutamates sont choisis parmi les acylglutamates mono- ou disodiques. Des composés convenant particulièrement bien aux compositions de la présente invention sont le stéaroyl glutamate monosodique, le myristoyl glutamate monosodique, le lauroyl glutamate monosodique et le stéaroyl glutamate disodique respectivement vendus sous les dénominations commerciales «Acylglutamate HS 11», «Acylglutamate MS 11», «Acylglutamate LS 11» et «Acylglutamate HS 21» par la société Ajinomoto.

Les acylglutamates utilisés pour la présente invention sont généralement présents dans les compositions de l'invention à une teneur supérieure à 0,2 % en poids par rapport au poids total de la composition, de préférence supérieure à 0,5%.

Une des caractéristiques essentielles des compositions visées par la présente invention est de contenir des nanopigments d'oxyde de titane. Par nanopigments, on entend des pigments dont la taille moyenne des particules élémentaires est comprise entre 5 et 100 nm De préférence, la taille moyenne des particules élémentaires des nanopigments visés par la présente invention est comprise entre 10 et 50 nm. De tels nanopigments d'oxyde de titane correspondent à des nanopigments d'oxyde de titane connus, habituellement utilisés dans le domaine cosmétique comme filtres, et qui peuvent être traités ou non traités.

L'oxyde de titane peut se présenter sous forme rutile, anatase ou amorphe, mais de préférence sous forme rutile et/ou anatase.

Les nanopigments traités peuvent par exemple être traités par l'alumine, la silice, les composés de l'aluminium, les composés du silicium, les composés du sodium les oxydes de fer, les esters de fer, l'acide stéarique, la glycérine.

Plus particulièrement, les nanopigments traités peuvent être des oxydes de titane traités par :

- la silice et l'alumine tels que les produits «MICROTITANIUM DIOXIDE MT 500 SA» et «MICROTITANIUM DIOXIDE MT 100 SA» de la société TAYCA, et les produits «TIOVEIL Fin», «TIOVEIL OP», «TIOVEIL MOTG» et «TIOVEIL IPM» de la société TIOXIDE,
- l'alumine et le stéarate d'aluminium tels que le produit «MICROTITANIUM DIOXIDE MT 100 T» de la société TAYCA,
- l'alumine et le laurate d'aluminium tels que le produit «MICROTITANIUM DIOXIDE MT 100 S» de la société TAYCA,
- des oxydes de fer et le stéarate de fer tels que le produit «MICROTITANIUM DIOXIDE MT 100 F» de la société TAYCA,
- la silice, l'alumine et la silicone tels que les produits «MICROTITANIUM DIOXIDE MT 100 SAS», «MICROTITANIUM DIOXIDE MT 600 SAS» et «MICROTITANIUM DIOXIDE MT 500 SAS» de la société TAYCA,
- l'hexamétaphosphate de sodium tels que le produit «MICROTITANIUM DIOXIDE MT 150 W» de la société TAYCA,
- l'octyltriméthoxysilane tels que le produit 《T-805 》de la société DEGUSSA,
- l'alumine et l'acide stéarique tels que le produit 《UVT-M160 》de la société KEMIRA,
- l'alumine et la glycérine tels que le produit 《UVT-M212 》de la société KEMIRA
- l'alumine et la silicone tels que le produit 《UVT-M262 》de la société KEMIRA.

Les oxydes de titane non traités peuvent par exemple être ceux vendus par la société TAYCA sous les dénominations commerciales 《MICROTITANIUM DIOXIDE MT 500 B 》ou 《MICROTITANIUM DIOXIDE MT 600 B 》.

Le ou les nanopigments d'oxyde de titane peuvent être présents dans la composition selon l'invention dans une proportion comprise entre 0,1 et 25% en poids par rapport au poids total de la composition, de préférence, entre 0,2 et 20% en poids par rapport au poids total de la composition.

Grâce à l'invention, on peut sans problème introduire des quantités relativement élevées de $TiO_2$, en particulier supérieures à 5% en poids, ou encore supérieures à 10% en poids, ou même supérieures à 15% en poids, et ceci sans être ou peu pénalisé par le problème du photobleuissement évoqué ci-avant.

Les compositions cosmétiques antisolaires selon l'invention peuvent bien entendu contenir un ou plusieurs filtres solaires actifs dans l'UVA et/ou l'UVB (absorbeurs), hydrophiles ou lipophiles. Ces filtres peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de $\beta,\beta$-diphénylacrylate, les dérivés de l'acide p-aminobenzoïque, les polymères filtres et silicones filtres décrits dans la demande WO-93/04665. Des exemples de filtres UVA qui peuvent être utilisés dans les compositions selon l'invention sont le butyl méthoxydibenzoylméthane vendu sous la dénomination commerciale 《Parsol 1789 》par la soviété Givaudan-Roure ou encore l'acide benzène 1,4-di(3-méthylidène-10-camphosulfonique) vendu sous la dénomination commerciale 《Mexoryl SX 》par la Demanderesse. Des exemples de filtres UVB qui peuvent être utilisés dans les compositions selon l'invention sont le 4-méthylbenzilidène camphre ou l'acide phénylbenzimidazole sulfonique respectivement vendus sous les dénominations commerciales 《Eusolex 6300 》et 《Eusolex 232 》par la société Rona/E.Merck, ou encore le 2-cyano 3,3-diphénylacrylate de 2-éthyl hexyle vendu sous la dénomination commerciale 《Uvinul N 539 》par la société BASF. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments autres que ceux mentionnés ci-avant. Ces pigments peuvent être des nanopigments d'oxydes métalliques enrobés ou non comme par exemple des nanopigments d'oxyde de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants et notamment les antioxydants ARL, les opacifiants, les stabilisants, les émollients, les silicones , les $\alpha$-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-$\alpha$-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (filtres, pigments, adjuvants, ...) et/ou leurs quantités de manière telle que les propriétés avantageuses attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel-crème, de poudre, de bâtonnet solide et éventuellement être conditionnée en aérosol et se présenter sous forme de mousse ou de spray.

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, ou encore des suspensions.

A titre indicatif, pour les formulations antisolaires conformes à l'invention qui présentent un support de type émulsion huile-dans-eau, la phase aqueuse (comprenant les filtres ou autres adjuvants hydrophiles) représente généralement de 50 à 95% en poids, de préférence de 70 à 90% en poids, par rapport à l'ensemble de la formulation, la phase huileuse (comprenant notamment le ou les filtres et autres adjuvants lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

De préférence, la composition selon l'invention se présente sous la forme d'une émulsion huile-dans-eau.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, illustrant l'invention vont maintenant être donnes.

**EXEMPLE 1 :**

On a réalisé des tests comparatifs afin de mettre en évidence l'amélioration apportée au niveau du photobleuissement par l'introduction d'acylglutamates au sein d'une composition contenant des nanopigments d'oxyde de titane.

On a réalisé quatre émulsions huile-dans-eau, chacune d'elles contenant un certain pourcentage x de stéaroyl glutamate monosodique vendu sous la dénomination commerciale 《 Acylglutamate HS 11 》par Ajinomoto et 5% d'oxyde de titane vendu sous la dénomination 《 MT 100 T 》par Tayca. En faisant varier x de 0 à 5%, on a ainsi réalisé quatre émulsions A, B, C et D. La composition du support commun (émulsion huile dans eau) était la suivante :

Support :

| | |
|---|---|
| - mélange de mono- et di-stéarate de glycérol / stéarate de polyéthylène glycol (100 OE) vendu sous la dénomination commerciale 《 Arlacel 165 》par ICI | 2 % |
| - benzoate d'alcools C12/C15 vendu sous la dénomination commerciale 《 Finsolv TN 》 | 8 % |
| - hydratant | 8 % |
| - dioxyde de titane vendu sous la dénomination commerciale 《 MT 100 T 》par Tayca | 5% |
| - stéaroyl glutamate monosodique vendu sous la dénomination commerciale 《 Acylglutamate HS 11 》par Ajinomoto | x % |
| - acide polyacrylique réticulé vendu sous la dénomination commerciale 《 Carbopol 980 》par Goodrich | 0,3 % |
| - agent pH | qs pH=7 |

(suite)

| | |
|---|---|
| - conservateurs | qs |
| - eau purifiée | qsp 100 % |

Pour ces quatre compositions, le photobleuissement a été évalué selon le protocole suivant : les compositions ont été introduites dans des boîtes en plastique transparentes aux UV ( boîtes polystyrène cristal 50x40x6 cm$^3$) et exposées aux UV (SUNTEST CPS Heraeus) pendant 12H. Les mesures colorimétriques ont été effectuées à l'aide d'un colorimètre Minolta CM1000 : une première mesure a été relevée juste avant l'exposition aux UV (T0) et une deuxième après douze heures d'exposition aux UV (T12H).

Les résultats sont exprimés dans le système (L, a, b) dans lequel L représente la luminance, a représente l'axe rouge-vert (-a = vert, +a = rouge) et b représente l'axe jaune-bleu (-b =bleu, +b =jaune).

Pour l'évaluation du photobleuissement, on s'intéresse à ΔE, qui mesure la variation de couleur totale. ΔE est calculé à partir des variations ΔL, Δa et Δb selon la formule suivante :

$$\Delta E = \sqrt{(\Delta L)^2 + (\Delta a)^2 + (\Delta b)^2}$$

où :

- Δb = b$_{T12H}$ - b$_{T0}$ et mesure la variation de la couleur bleue,
- ΔL = L$_{T12H}$ - L$_{T0}$ et mesure le noircissement de la composition.

Plus ΔE est faible, plus la protection contre le photobleuissement est efficace.
Les résultats sont consignés dans le tableau (I) suivant :

Tableau (I)

| Formule | x% | ΔE |
|---|---|---|
| A (comparatif) | 0 | 32 |
| B (invention) | 0,2 | 27,4 |
| C (invention) | 0,5 | 23,9 |
| D (invention) | 5 | 22,1 |

Ces résultats montrent clairement que l'introduction d'acylglutamate au sein d'une composition contenant des oxydes de titane diminue significativement le photobleuissement observé habituellement sur de telles compositions.

**EXEMPLE 2 :**

On a également réalisé trois émulsions huile-dans-eau, respectivement E, F et G, chacune d'elles contenant 1% d'acylglutamate et 5% d'oxyde de titane vendu sous la dénomination 〈〈MT 100T 〉〉par Tayca. Le support commun de ces trois émulsions est le même que celui utilisé à l'exemple 1, dans lequel on a fait varier la nature de l'acylglutamate. La composition E contient 1% de myristoyl glutamate monosodique vendu sous la dénomination commerciale 〈〈Acylglutamate MS 11 〉〉par Ajinomoto; la composition F contient 1 % de lauroyl glutamate monosodique vendu sous la dénomination commerciale 〈〈Acylglutamate LS 11 〉〉par Ajinomoto; et la composition G contient 1% de stéaroyl glutamate disodique vendu sous la dénomination commerciale 〈〈Acylglutamate HS 21 〉〉par Ajinomoto.

Les résultats, obtenus selon le même protocole que pour l'exemple 1 ci-dessus sont consignés dans le tableau (II) suivant :

Tableau (II)

| Formule | Acylglutamate | ΔE |
|---|---|---|
| A (comparatif) | pas d'acylglutamate | 32 |
| E (invention) | 〈〈Acylglutamate MS 11 〉〉 | 24,4 |

Tableau (II) (suite)

| Formule | Acylglutamate | ΔE |
|---|---|---|
| F (invention) | 《Acylglutamate LS 11》 | 24,1 |
| G (invention) | 《Acylglutamate HS 21》 | 24,6 |

Ces résultats montrent bien que l'introduction d'un acylglutamate, quelle que soit sa nature, diminue le photobleuissement attaché à la présence d'oxyde de titane.

**EXEMPLE 3 :**

L'émulsion huile-dans-eau suivante donne un exemple concret d'une composition solaire conforme à l'invention. Les quantités sont exprimées en % de poids par rapport au poids total de la composition :

Phase A :

| | |
|---|---|
| - mélange de mono- et di-stéarate de glycérol / stéarate de polyéthylène glycol (100 OE) vendu sous la dénomination commerciale 《Arlacel 165》 par ICI | 2 % |
| - acide stéarique vendu sous la dénomination commerciale 《Stéarine TP》 par Stéarinerie Dubois | 2,5 % |
| - benzoate d'alcools C12/C15 vendu sous la dénomination commerciale 《Finsolv TN》 | 8 % |
| - triéthanolamine | 0,45 % |

Phase B :

| | |
|---|---|
| - glycérine | 8 % |
| - dioxyde de titane vendu sous la dénomination commerciale 《MT 100 T》 par Tayca | 5% |
| - stéaroyl glutamate monosodique vendu sous la dénomination commerciale 《Acylglutamate HS 11》 par Ajinomoto | 0,5 % |

Phase C :

| | |
|---|---|
| - acide polyacrylique réticulé vendu sous la dénomination commerciale 《Carbopol 980》 par Goodrich | 0,3 % |
| - cyclopentadiméthylsiloxane vendu sous la dénomination commerciale 《DC 245 Fluid》 | 5 % |

Phase D :

| | |
|---|---|
| - agent pH | qs pH=7 |

Phase E :

| - conservateurs | qs |
|---|---|
| - eau purifiée | qsp 100 % |

La composition ci-dessus a été réalisée de la façon suivante : on a porté la phase A à 80-85°C sous agitation. On a ensuite porté la phase B et une partie de l'eau à 80-85°C sous agitation. Puis on a versé la phase A sur la phase B sous agitation pour réaliser l'émulsion. On a refroidi le mélange vers 45°C, puis on a ajouté la phase C préalablement dispersée. On a neutralisé le gel. On a poursuivi le refroidissement et on a finalement ajouté les conservateurs et le solde de l'eau.

**Revendications**

1. Composition cosmétique à usage topique, en particulier pour la photoprotection de la peau et/ou des cheveux, caractérisée par le fait qu'elle est exempte de vésicules et qu'elle comprend dans un support cosmétiquement acceptable, au moins un nanopigment d'oxyde de titane et au moins un lipide amphiphile ionique choisi parmi les acylaminoacides.

2. Composition selon la revendication 1, caractérisée par le fait que le lipide amphiphile ionique est choisi parmi les acylglutamates.

3. Composition selon la revendication 2, caractérisée par le fait que les acylglutamates comprennent une chaîne acyle allant de 10 à 30 atomes de carbone.

4. Composition selon la revendication 3, caractérisée par le fait que les acylglutamates comprennent une chaîne acyle allant de 10 à 20 atomes de carbone.

5. Composition selon l'une quelconque des revendications 2 à 4, caractérisée par le fait que le lipide amphiphile ionique est choisi parmi les acylglutamates monosodiques et disodiques.

6. Composition selon la revendication 5, caractérisée par le fait que l'acylglutamate est choisi parmi le stéaroyl glutamate monosodique, le myristoyl glutamate monosodique, le lauroyl glutamate monosodique et le stéaroyl glutamate disodique.

7. Composition selon l'une quelconque des revendications précédentes, caractérisée par le fait que le lipide amphiphile ionique est présent dans une teneur supérieure à 0,2 % en poids par rapport au poids total de la composition.

8. Composition selon la revendication 7, caractérisée par le fait que ladite teneur est supérieure à 0,5 % en poids par rapport au poids total de la composition.

9. Composition selon l'une quelconque des revendications précédentes, caractérisée par le fait que le nanopigment d'oxyde de titane est présent à une teneur allant de 0,1 à 25 % en poids par rapport au poids total de la composition.

10. Utilisation d'une composition telle que définie à l'une des revendications 1 à 9 comme, ou pour la fabrication de, compositions cosmétiques destinées à la protection de la peau et/ou des cheveux contre le rayonnement ultraviolet, en particulier le rayonnement solaire.

11. Procédé de traitement cosmétique pour la protection de la peau et/ou des cheveux contre le rayonnement ultraviolet, en particulier le rayonnement solaire, consistant à appliquer sur ces derniers une quantité efficace d'une composition telle que définie à l'une quelconque des revendications 1 à 9.

12. Utilisation d'un acylaminoacide, dans une composition contenant des pigments d'oxyde de titane, pour diminuer le photobleuissement dû à la présence desdits pigments.

**13.** Utilisation selon la revendication 12, caractérisée par le fait que l'acylaminoacide est un acylglutamate.

**14.** Utilisation selon la revendication 13, caractérisée par le fait que ledit acylglutamate est tel que défini à l'une quelconque des revendications 3 à 6.

**15.** Procédé pour réduire le photobleuissement d'une composition contenant des pigments d'oxyde de titane, consistant à introduire dans la composition au moins un acylaminoacide.

**16.** Procédé selon la revendication 15, caractérisé par le fait que l'acylaminoacide est un acylglutamate.

**17.** Procédé selon la revendication 16, caractérisé par le fait que ledit acylglutamate est tel que défini à l'une quelconque des revendications 3 à 6.

**Claims**

**1.** Cosmetic composition for topical use, in particular for the photoprotection of the skin and/or hair, characterized in that it does not contain vesicles and in that it comprises, in a cosmetically acceptable vehicle, at least one titanium oxide nanopigment and at least one ionic amphiphilic lipid chosen from acylamino acids.

**2.** Composition according to Claim 1, characterized in that the ionic amphiphilic lipid is chosen from acylglutamates.

**3.** Composition according to Claim 2, characterized in that the acylglutamates comprise an acyl chain ranging from 10 to 30 carbon atoms.

**4.** Composition according to Claim 3, characterized in that the acylglutamates comprise an acyl chain ranging from 10 to 20 carbon atoms.

**5.** Composition according to any one of Claims 2 to 4, characterized in that the ionic amphiphilic lipid is chosen from monosodium and disodium acylglutamates.

**6.** Composition according to Claim 5, characterized in that the acylglutamate is chosen from monosodium stearoylglutamate, monosodium myristoylglutamate, monosodium lauroylglutamate and disodium stearoylglutamate.

**7.** Composition according to any one of the preceding claims, characterized in that the ionic amphiphilic lipid is present in an amount greater than 0.2% by weight with respect to the total weight of the composition.

**8.** Composition according to Claim 7, characterized in that the said content is greater than 0.5% by weight with respect to the total weight of the composition.

**9.** Composition according to any one of the preceding claims, characterized in that the titanium oxide nano-pigment is present at a content ranging from 0.1 to 25% by weight with respect to the total weight of the composition.

**10.** Use of a composition as defined in one of Claims 1 to 9 as, or for the manufacture of, cosmetic compositions intended for the protection of the skin and/or hair against ultraviolet radiation, in particular solar radiation.

**11.** Cosmetic treatment process for the protection of the skin and/or hair against ultraviolet radiation, in particular solar radiation, which comprises the application to the latter of an effective amount of a composition as defined in any one of Claims 1 to 9.

**12.** Use of an acylamino acid in a composition containing titanium oxide pigments, in order to decrease the photoblueing due to the presence of the said pigments.

**13.** Use according to Claim 12, characterized in that the acylamino acid is an acylglutamate.

**14.** Use according to Claim 13, characterized in that the said acylglutamate is as defined in any one of Claims 3 to 6.

**15.** Process for reducing the photoblueing of a composition containing titanium oxide pigments, which comprises the introduction into the composition of at least one acylamino acid.

16. Process according to Claim 15, characterized in that the acylamino acid is an acylglutamate.

17. Process according to Claim 16, characterized in that the said acylglutamate is as defined in any one of Claims 3 to 6.

**Patentansprüche**

1. Kosmetische Zusammensetzungen zur topischen Anwendung, insbesondere zum Schutz der Haut und/oder der Haare gegen Licht, dadurch gekennzeichnet, daß sie keine Vesikel aufweisen und in einem kosmetisch akzeptablen Träger mindestens ein Nanopigment von Titandioxid und mindestens ein ionisches amphiphiles Lipid, das unter den Acylaminosäuren ausgewählt ist, enthalten.

2. Zusammensetzungen nach Anspruch 1, dadurch gekennzeichnet, daß das ionische amphiphile Lipid unter den Acylglutamaten ausgewählt ist.

3. Zusammensetzungen nach Anspruch 2, dadurch gekennzeichnet, daß die Acylglutamate eine Acylkette mit 10 bis 30 Kohlenstoffatomen aufweisen.

4. Zusammensetzungen nach Anspruch 3, dadurch gekennzeichnet, daß die Acylglutamate eine Acylkette mit 10 bis 20 Kohlenstoffatomen aufweisen.

5. Zusammensetzungen nach einem der Ansprüche 2 bis 4, dadurch gekennzeichnet, daß das ionische amphiphile Lipid unter den Mononatriumacylglutamaten und Dinatriumacylglutamaten ausgewählt ist.

6. Zusammensetzungen nach Anspruch 5, dadurch gekennzeichnet, daß das Acylglutamat unter Mononatriumstearoylglutamat, Mononatriummyristoylglutamat, Mononatriumlauroylglutamat und Dinatriumstearoylglutamat ausgewählt ist.

7. Zusammensetzungen nach einem der vorhergehenden Ansprüche, dadurch gekennzeichnet, daß das ionische amphiphile Lipid in einem Mengenanteil über 0,2 Gew.-%, bezogen auf das Gesamtgewicht der Zusammensetzung, vorliegt.

8. Zusammensetzungen nach Anspruch 7, dadurch gekennzeichnet, daß dieser Mengenanteil über 0,5 Gew.-%, bezogen auf das Gesamtgewicht der Zusammensetzung, liegt.

9. Zusammensetzungen nach einem der vorhergehenden Ansprüche, dadurch gekennzeichnet, daß das Nanopigment von Titandioxid in einem Mengenanteil von 0,1 bis 25 Gew.-%, bezogen auf das Gesamtgewicht der Zusammensetzung, vorliegt.

10. Verwendung von Zusammensetzungen nach einem der Ansprüche 1 bis 9 als kosmetische Zusammensetzungen, die zum Schutz der Haut und/oder der Haare gegen ultraviolette Strahlung, insbesondere gegen Sonnenstrahlung, bestimmt sind, oder zu deren Herstellung.

11. Verfahren zur kosmetischen Behandlung, um die Haut und/oder die Haare gegen ultraviolette Strahlung, insbesondere gegen Sonnenstrahlung, zu schützen, das darin besteht, auf die Haut und/oder die Haare eine wirksame Menge einer Zusammensetzung nach einem der Ansprüche 1 bis 9 aufzutragen.

12. Verwendung einer Acylaminosäure in Zusammensetzungen, die Titandioxidpigmente enthalten, zur Verminderung der durch Licht ausgelösten Blaufärbung, die von der Anwesenheit dieser Pigmente rührt.

13. Verwendung nach Anspruch 12, dadurch gekennzeichnet, daß die Acylaminosäure ein Acylglutamat ist.

14. Verwendung nach Anspruch 13, dadurch gekennzeichnet, daß dieses Acylglutamat ein in einem der Ansprüche 3 bis 6 definiertes Acylglutamat ist.

15. Verfahren zur Verminderung der durch Licht ausgelösten Blaufärbung von Zusammensetzungen, die Titandioxidpigmente enthalten, das darin besteht, in die Zusammensetzungen mindestens eine Acylaminosäure einzubringen.

16. Verfahren nach Anspruch 15, dadurch gekennzeichnet, daß die Acylaminosäure ein Acylglutamat ist.

17. Verfahren nach Anspruch 16, dadurch gekennzeichnet, daß dieses Acylglutamat ein in einem der Ansprüche 3 bis 6 definiertes Acylglutamat ist.